# EUROPEAN PATENT APPLICATION

(11) **EP 4 652 929 A1**
(43) Date of publication of application: **26.11.2025**
(21) Application number: 25174838.0
(22) Date of filing: 07.05.2025
(51) Int. Cl.: A61B 5/145, A61B 5/1486, A61B 5/1495, A61B 5/00

(54) **ELECTRONIC APPARATUS AND METHOD OF CONTROLLING THE SAME**

(30) Priority: 22.05.2024 KR 20240066588
(71) Applicant: I-SENS, INC., Seoul 06646 (KR)
(72) Inventor: Seo, Jung Hee, 06646 Seoul (KR); Mok, Jin Won, 06646 Seoul (KR)
(74) Representative: Westphal, Mussgnug & Partner Patentanwälte mbB

(57) **Abstract**

Provided are an electronic apparatus and a method of controlling the same. The method includes receiving a sensor data related to a concentration of an analyte from an analyte monitoring device at least partially implantable beneath a skin of a user; displaying a UI element for receiving a calibration information from the user, wherein the concentration of the analyte is acquired based on the sensor data and the calibration information; and deactivating the UI element when an error related to the sensor data is detected.

## Description

### CROSS-REFERENCE TO RELATED APPLICATION

This application claims priority to and the benefit of Korean Patent Application No. 10-2024-0066588, filed on May 22, 2024, the disclosure of which is incorporated herein by reference in its entirety.

### BACKGROUND

### 1. Field of the Invention

The present disclosure relates to an electronic apparatus and a method for controlling the same, and more particularly, to an electronic apparatus for providing a concentration of an analyte to a user and a method for controlling the same.

### 2. Discussion of Related Art

A Continuous Glucose Monitoring System (CGMS) is a system that acquires a user's blood glucose value using a sensor in contact with the user's body fluid (e.g., interstitial fluid) and provides it to the user. The continuous glucose monitoring system includes a continuous glucose monitor that detects a sensor signal related to the concentration of glucose from the user's body fluid, and a user terminal device that acquires and provides the blood glucose concentration to the user based on the sensor signal.

Meanwhile, there may be cases where the sensor signal does not reflect the actual blood glucose value. For example, when excessive pressure is applied to the body part where the continuous glucose monitor is attached, the blood glucose value derived through the sensor signal may be significantly lower than the actual blood glucose value. Alternatively, due to sensor instability, there may be cases where the rate of change of the blood glucose value derived through the sensor signal increases excessively. In this way, providing inaccurate blood glucose values to the user in situations where the sensor signal does not reflect the actual blood glucose value (i.e., situations where there is an error in the sensor signal) may cause confusion to the user.

Therefore, there is a need for a method to control an electronic apparatus to prevent user confusion when there is an error in the sensor signal.

### SUMMARY OF THE INVENTION

The present disclosure is directed to providing an electronic apparatus that detects an error in a sensor signal.

The present disclosure is also directed to providing an electronic apparatus that prevents inaccurate blood glucose values from being provided to a user according to an error in the sensor signal.

Technical objects of the present disclosure are not limited to those described above, and other technical objects that have not been described above will be clearly understood by those of ordinary skill in the art from the following description.

According to one embodiment of the present disclosure, a method for controlling an electronic apparatus may be provided, including: receiving a sensor data related to a concentration of an analyte from an analyte monitoring device at least partially implantable beneath a skin of a user; displaying a UI element for receiving a calibration information from the user, wherein the concentration of the analyte is acquired based on the sensor data and the calibration information; and deactivating the UI element when an error related to the sensor data is detected.

The error may be detected when a rate of change of the concentration of the analyte is outside a preset range.

The error may be detected when a rate of change of the concentration of the analyte remains below a threshold value for a preset period.

The threshold value may be adjusted based on whether the user is sleeping.

The error may not be detected when the user is not sleeping.

The error may not be detected when a time point corresponding to the concentration of the analyte does not belong to a predetermined time period.

The method may further include: acquiring the concentration of the analyte by calibrating the sensor data based on the calibration information when the calibration information is received from the user.

The concentration of the analyte may be displayed in a first display mode when the error is detected, and the concentration of the analyte may be displayed in a second display mode different from the first display mode when the error is not detected.

The method may further include: modifying a first concentration of the analyte corresponding to a first time point based on a second concentration of the analyte corresponding to a second time point around the first time point, when an error related to a first sensor data corresponding to the first concentration of the analyte is detected.

The UI element may be displayed simultaneously with the concentration of the analyte on the same screen.

According to another embodiment of the present disclosure, an electronic apparatus may be provided, including: a display; a communication interface including at least one communication circuit; a memory storing at least one instruction; and a processor; wherein the processor, by executing the at least one instruction, receives a sensor data related to a concentration of an analyte from an analyte monitoring device at least partially implantable beneath a skin of a user, displays a UI element on the display for receiving a calibration information from the user, wherein the concentration of the analyte is acquired based on the sensor data and the calibration information is used for calibrating the sensor data, and deactivates the UI element when an error related to the concentration of the analyte is detected.

The processor may detect the error when a rate of change of the concentration of the analyte is outside a preset range.

The processor may detect the error when a rate of change of the concentration of the analyte remains below a threshold value for a preset period.

The processor may adjust the threshold value based on whether the user is sleeping.

The processor may not detect the error when the user is not sleeping.

The processor may not detect the error when a time point corresponding to the concentration of the analyte does not belong to a predetermined time period.

The processor may acquire the concentration of the analyte by calibrating the sensor data based on the calibration information when the calibration information is received from the user.

The processor may display the concentration of the analyte in a first display mode when the error is detected, and may display the concentration of the analyte in a second display mode different from the first display mode when the error is not detected.

The processor may modify a first concentration of the analyte corresponding to a first time point based on a second concentration of the analyte corresponding to a second time point around the first time point, when an error related to the first concentration of the analyte is detected.

The processor may display the UI element simultaneously with the concentration of the analyte on the same screen.

The means for solving the problems of the present disclosure are not limited to the solutions described above, and means not mentioned may be clearly understood by those skilled in the art to which the present disclosure pertains from the specification and the accompanying drawings.

### BRIEF DESCRIPTION OF THE DRAWINGS

Aspects, features, and advantages of the present disclosure will become more apparent from the following detailed description when taken in conjunction with the accompanying drawings.
FIG 1 is a schematic diagram illustrating a continuous glucose monitoring system according to an embodiment of the present disclosure.
FIG 2 is a flowchart illustrating a method for controlling an electronic apparatus according to an embodiment of the present disclosure.
FIG 3 is a graph illustrating blood glucose values according to an embodiment of the present disclosure.
FIG 4 is a graph illustrating blood glucose rate of change according to an embodiment of the present disclosure.
FIG 5 is a diagram for explaining a blood glucose display screen according to an embodiment of the present disclosure.
FIG 6 is a schematic diagram illustrating a blood glucose display method according to an embodiment of the present disclosure.
FIG 7 is a schematic diagram illustrating a blood glucose display method according to another embodiment of the present disclosure.
FIG 8 is a block diagram illustrating the configuration of an analyte monitoring system (1000) according to an embodiment of the present disclosure.

### DETAILED DESCRIPTION OF EXEMPLARY EMBODIMENTS

Terminology used in the present specification will be briefly described first, and then the present disclosure will be described in detail.

As terms used herein, general terms currently used as widely as possible will be selected in consideration of functionality in the present disclosure, but may vary depending on the intent of those of ordinary skill in the art, precedents, the advent of new technology, and the like. In particular, a term may be arbitrarily selected by the applicant. In this case, the meaning of the term will be explained in detail through the relevant description of the disclosure. Therefore, the terms used herein should be defined on the basis of their meanings and the overall content of the present disclosure rather than their names.

The present disclosure may be modified in various ways and have various embodiments, and specific embodiments will be illustrated in the drawings and described in detail. However, this does not intend to limit the present disclosure to specific embodiments, and it is to be understood that the present disclosure includes all modifications, equivalents, and substitutions within the disclosed spirit and technical scope. In describing embodiments, a detailed description of relevant known technology will be omitted when determined to obscure the subject matter of the present disclosure.

Terms such as "first," "second," and the like may be used to describe various components, but components are not limited by the terms. The terms are only used for the purpose of distinguishing one component from others.

Singular expressions include plural expressions unless the context clearly indicates otherwise. In this application, the terms "include," "have," and the like indicate the presence of features, integers, steps, operations, components, parts, or combinations thereof described in the present specification and do not preclude the presence or addition of one or more other features, integers, steps, operations, components, parts, or combinations thereof.

Hereinafter, embodiments of the present disclosure will be described in detail with reference to the accompanying drawings so that those skilled in the technical field to which the present disclosure pertains can easily implement the present disclosure. However, the present disclosure can be implemented in many different forms and is not limited to the embodiments described herein. To clearly describe the present disclosure, parts irrelevant to the description will be omitted in the drawings, and throughout the specification, like reference numerals refer to like parts.

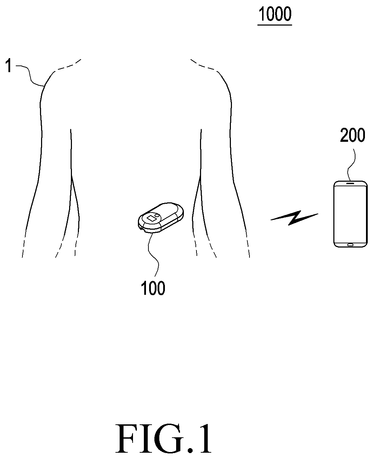

FIG 1 is a schematic diagram illustrating a continuous glucose monitoring system according to an embodiment of the present disclosure.

Referring to FIG 1, the analyte monitoring system (1000) may include an analyte monitoring device (100) and an electronic apparatus (200). For example, the analyte monitoring system (1000) may be a Continuous Glucose Monitoring System (CGMS), and the analyte monitoring device (100) may be a Continuous Glucose Monitor (CGM). The electronic apparatus (200) may be a user terminal device. For example, the electronic apparatus (200) may be a smartphone, a tablet PC, a smart watch, a PDA, or a dedicated receiver (e.g., a Receiver).

The analyte monitoring device (100) may include an analyte sensor insertable into the body of a user (1). The analyte monitoring device (100) may acquire sensor data related to the concentration of an analyte contained in the user's (1) body fluid (e.g., interstitial fluid) through the analyte sensor. The sensor data may include a sensor signal (e.g., a current signal) measured by the analyte sensor. The analyte may include glucose and ketone.

The analyte monitoring device (100) may be communicatively connected to the electronic apparatus (200). For example, the analyte monitoring device (100) may be connected to the electronic apparatus (200) according to a Bluetooth protocol. The analyte monitoring device (100) may transmit sensor data to the electronic apparatus (200) according to a predefined schedule (e.g., every 5 minutes).

The electronic apparatus (200) may acquire the concentration of the analyte based on the sensor data received from the analyte monitoring device (100). The electronic apparatus (200) may derive the concentration of the analyte by calibrating the sensor data based on calibration information. The calibration information may include the user's (1) blood glucose value measured through a blood glucose meter. For example, the user (1) may input their blood glucose value measured with a blood glucose meter into the electronic apparatus (200).

The electronic apparatus (200) may provide the concentration of the analyte to the user (1). For example, the electronic apparatus (200) may display a chart or graph representing the concentration of the analyte on a display. Accordingly, the user (1) can check the concentration of the analyte in real-time through the electronic apparatus (200).

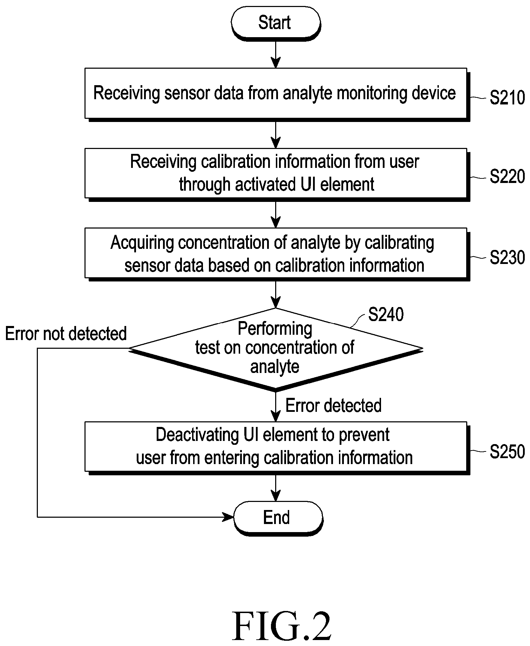

FIG 2 is a flowchart illustrating a method for controlling an electronic apparatus according to an embodiment of the present disclosure.

Referring to FIG 2, the electronic apparatus (200) may receive sensor data from the analyte monitoring device (100) (S210). The electronic apparatus (200) may receive sensor data from the analyte monitoring device (100) according to a predefined schedule (e.g., every 5 minutes). The electronic apparatus (200) may maintain a communication connection with the analyte monitoring device (100) at all times, or establish a communication connection at predefined time intervals (e.g., every 5 minutes).

The electronic apparatus (200) may receive calibration information from the user through an activated UI element (S220). The UI element may be displayed on a display equipped on the electronic apparatus (200). The user may input calibration information through the UI element. The calibration information is information for calibrating the sensor data, for example, it may be a blood glucose value measured through a blood glucose meter.

The electronic apparatus (200) may acquire the concentration of the analyte by calibrating the sensor data based on the calibration information (S230). In this disclosure, calibration may be a process of deriving the concentration of the analyte from the sensor data using the calibration information. The electronic apparatus (200) may calibrate the sensor data using a predefined algorithm (e.g., linear regression).

The electronic apparatus (200) may perform a test on the concentration of the analyte (S240). The test (or self-diagnostic test) may be a procedure for determining whether there is an abnormality (or error) in the concentration of the analyte. In one embodiment, the electronic apparatus (200) may detect whether the rate of change of the concentration of the analyte is outside a preset range. The preset range may be from -3 (mg/dL/min) to +3 (mg/dL/min). In another embodiment, the electronic apparatus (200) may detect whether the rate of change of the concentration of the analyte is maintained below a threshold value for a preset period. The preset period may be 20 minutes. As another embodiment of the test, the electronic apparatus (200) may detect whether the sensitivity of the analyte sensor (110) is maintained below a threshold value for more than a preset period.

According to various embodiments of the present disclosure, the electronic apparatus (200) may detect an error by sensing a pattern where the concentration of the analyte is stably maintained for a certain period and then suddenly drops. This abnormal concentration pattern may occur mainly due to physical pressure applied to the analyte sensor (110), which temporarily reduces the sensitivity of the sensor. When the sensitivity of the analyte sensor (110) decreases, a signal with a smaller magnitude than the ideal signal may be measured, resulting in a value lower than the actual analyte concentration. This phenomenon may frequently occur especially when the user takes a posture that compresses the body part (e.g., upper arm) where the analyte sensor (110) is attached while sleeping.

In addition, the electronic apparatus (200) may analyze time series data of the analyte concentration for this pattern detection, and apply specific criteria such as cases where the rate of change of the concentration over a certain period (e.g., 30 minutes) is outside the normal range (e.g., decreases by more than 20% within 5 minutes). Through this, the electronic apparatus (200) can distinguish between errors due to physical pressure on the sensor and concentration changes due to actual physiological changes.

The electronic apparatus (200) may detect an error based on whether the user is sleeping. In one embodiment, the electronic apparatus (200) may not detect an error when the user is not sleeping. **In** this case, the error may be detected only when the user is sleeping. This may be because errors in sensor data mainly occur when the user is sleeping. More specifically, errors may occur when the user takes a posture that excessively compresses the body part where the analyte sensor is attached while sleeping.

**In** another embodiment, even if the user is not sleeping, an error may be detected, but the threshold value for error detection may be set differently depending on the user's sleep status. For example, when the user is not sleeping, the threshold value may be -3 (mg/dL/min), and when the user is sleeping, the threshold value may be -2 (mg/dL/min). **In** this case, if the concentration of the analyte is less than the threshold value, an error may be detected. When the concentration of the analyte is - 2.5 (mg/dL/min), an error may not be detected when the user is not sleeping, but may be detected when the user is sleeping.

The electronic apparatus (200) may set a different preset range for error detection depending on the user's sleep status. In this case, if the concentration of the analyte is outside the preset range, an error may be detected. For example, when the user is not sleeping, the preset range may be from -3 (mg/dL/min) to +3 (mg/dL/min), and when sleeping, the preset range may be from -2 (mg/dL/min) to +2 (mg/dL/min). When the concentration of the analyte is 2.5 (mg/dL/min), an error may not be detected when the user is not sleeping, but may be detected when the user is sleeping. That is, in a non-sleep state, as the normal range becomes wider than in a sleep state, the probability of error detection may decrease.

The electronic apparatus (200) may identify the user's sleep status by receiving information indicating the user's sleep state through a wearable device. Additionally, the electronic apparatus (200) may determine the user's sleep status by referring to a memo stored in the electronic apparatus (200) that the user has recorded. For example, if the user has written their sleep time as a memo, the electronic apparatus (200) may determine the user's sleep status based on the memo and the local time.

Furthermore, the electronic apparatus (200) may detect errors based on other information related to sleep status (e.g., time). For example, if the time point corresponding to the concentration of the analyte belongs to a predetermined time interval, the electronic apparatus (200) may detect errors in the same way as when the user is sleeping. If the time point corresponding to the concentration of the analyte does not belong to a predetermined time interval, the electronic apparatus (200) may detect errors in the same way as when the user is not sleeping. That is, the predetermined time interval may correspond to the user's sleep time.

If an error is detected, the electronic apparatus (200) may deactivate the UI element so that the user cannot input calibration information (S250). When calibration information is input while there is an error in the sensor data, an inaccurate concentration of the analyte may be acquired. Therefore, the electronic apparatus (200) may prevent the acquisition of an inaccurate concentration of the analyte by deactivating the UI element. The electronic apparatus (200) may change or stop the display of the UI element. If no error is detected, the electronic apparatus (200) may maintain the activation of the UI element. Therefore, the user can input calibration information through the UI element. Meanwhile, even if an error is detected, the electronic apparatus (200) may acquire the concentration of the analyte based on the calibration information previously input by the user.

The electronic apparatus (200) may acquire the concentration of the analyte based on new sensor data received after the error is detected, and perform a test on the acquired concentration of the analyte. If an error is no longer detected, the electronic apparatus (200) may reactivate the UI element. Also, the electronic apparatus (200) may modify the concentration of the analyte at the time point where the error exists based on the concentration of the analyte at the time point where there is no error. Alternatively, the electronic apparatus (200) may derive the concentration of the analyte at the time point where the error exists based on the concentration of the analyte at the time point where there is no error.

For example, a first concentration may be acquired based on the sensor data at a first time point, a second concentration may be acquired based on the sensor data at a second time point after the first time point, and a third concentration may be acquired based on the sensor data at a third time point after the second time point. As a test result, no error may be detected for the first concentration and the third concentration, but an error may be detected for the second concentration. In other words, the sensor data at the first time point and the third time point may be normal, but the sensor data at the second time point may be abnormal.

In this case, the electronic apparatus (200) may newly calculate the concentration at the second time point based on the first concentration and the third concentration. For example, the electronic apparatus (200) may calculate a new concentration at the second time point by performing interpolation on the first concentration and the third concentration. Alternatively, the electronic apparatus (200) may calculate a new concentration at the second time point by performing extrapolation on the first concentration. Also, the electronic apparatus (200) may modify the second concentration based on the first concentration and the third concentration.

The electronic apparatus (200) may display the concentration of the analyte based on error detection. The electronic apparatus (200) may differently display a first concentration acquired based on normal first sensor data and a second concentration acquired based on abnormal second sensor data. For example, the first concentration and the second concentration may be displayed with different shapes, forms, and/or colors.

The electronic apparatus (200) may temporarily not display the concentration of the analyte during a time interval where there is an error. For example, the electronic apparatus (200) may not display the second concentration acquired based on the sensor data at the second time point where the error exists. Therefore, a data gap may be displayed at the second time point. The electronic apparatus (200) may fill in the data gap with the newly acquired or modified second concentration as the error disappears. In another embodiment, the electronic apparatus (200) may permanently not display the concentration of the analyte during a time interval where there is an error.

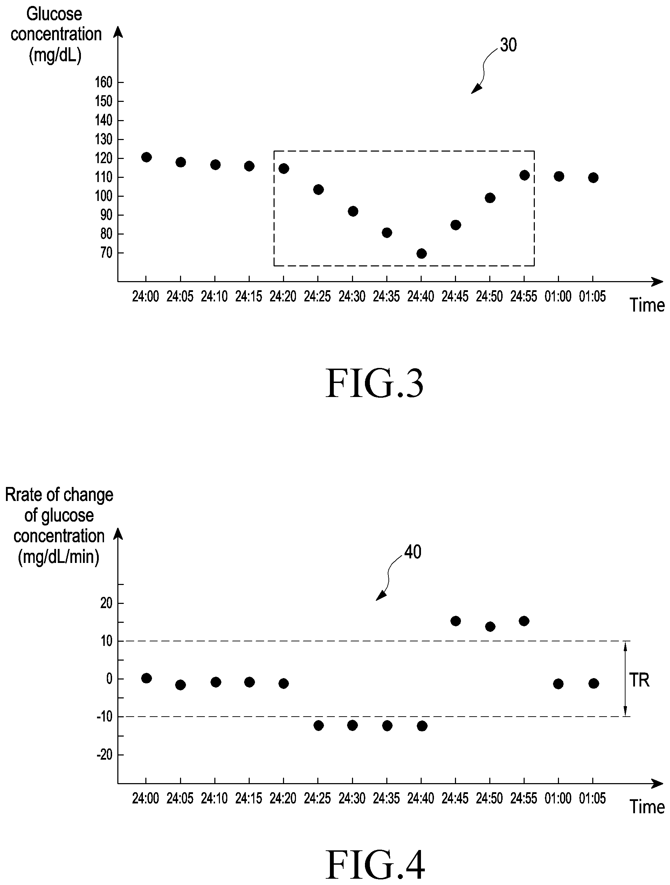

FIG 3 is a graph illustrating blood glucose values according to an embodiment of the present disclosure.

Referring to FIG 3, the values shown in the graph (30) may be blood glucose values calculated by the electronic apparatus (200) based on sensor data received from the analyte monitoring device (100) worn by the user (1). The x-axis of the graph (30) represents time and the y-axis represents the concentration of glucose (i.e., blood glucose value).

Referring to the graph (30), it can be seen that the blood glucose value decreases rapidly from 24:20 to 24:40 and increases rapidly from 24:40 to 24:55. However, the blood glucose values from 24:25 to 24:50 may differ from the user's (1) actual blood glucose values. For example, the blood glucose values from 24:25 to 24:50 may have been calculated based on inaccurately measured sensor signals due to pressure applied to the body part where the sensor is worn while the user (1) was asleep. When pressure is applied to the sensor wearing site, it may affect the movement of glucose in the vicinity of the site, resulting in a sensor signal with a smaller magnitude compared to a sensor signal corresponding to the user's actual blood glucose value.

The electronic apparatus (200) may calculate the rate of change of the blood glucose value (i.e., blood glucose rate of change) based on the sensor data. The electronic apparatus (200) may perform a test on the blood glucose value based on the blood glucose rate of change to detect an error. The electronic apparatus (200) may detect whether the magnitude of the blood glucose rate of change is maintained above a threshold value (e.g., 10mg/dL/min) for a preset period (e.g., 20 minutes). In the graph (30), the magnitude of the blood glucose rate of change may be above the threshold value from 24:25 to 24:55 (i.e., for 30 minutes). Specifically, the blood glucose rate of change from 24:25 to 24:40 may be less than -10(mg/dL/min), and the blood glucose rate of change from 24:45 to 24:55 may be greater than 10(mg/dL/min). In this case, the electronic apparatus (200) may determine that an error related to the blood glucose value has been detected.

The electronic apparatus (200) may detect an error considering the user's (1) sleep status. For example, if the user (1) is not sleeping between 24:20 and 24:55, the electronic apparatus (200) may determine that no error has been detected. On the other hand, if the user (1) is sleeping between 24:20 and 24:55, the electronic apparatus (200) may determine that an error has been detected.

The electronic apparatus (200) may determine an error by referring to the blood glucose value input by the user in addition to the blood glucose value calculated based on the sensor data. For example, if the difference between the calculated blood glucose value and the input blood glucose value that temporally correspond exceeds a predefined range, the electronic apparatus (200) may determine that an error has been detected. On the other hand, if the difference between the calculated blood glucose value and the input blood glucose value that temporally correspond is within a predefined range, the electronic apparatus (200) may determine that no error has been detected.

The electronic apparatus (200) may detect an error by referring to the past blood glucose trend as of the current point in time. The electronic apparatus (200) may determine an error by considering whether the past blood glucose value is temporarily abnormal while being normally normal. For example, if the past blood glucose value is also abnormal, the electronic apparatus (200) may determine that the current blood glucose value is due to a cause other than pressure applied to the sensor wearing site.

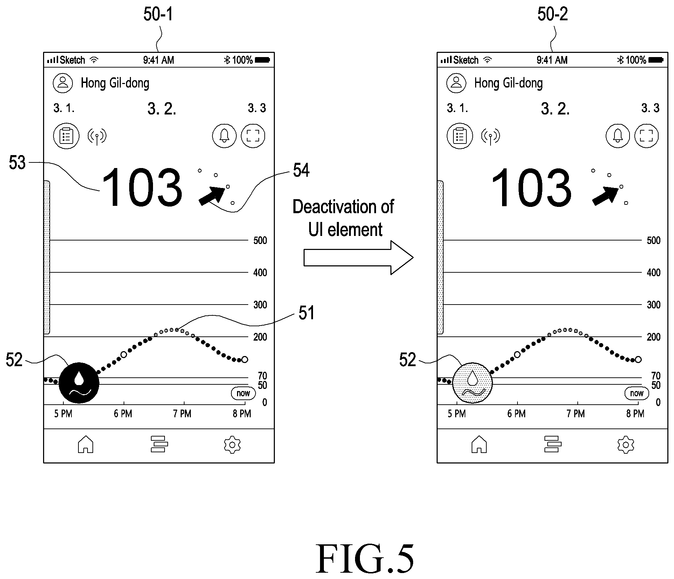

If an error is detected, the electronic apparatus (200) may deactivate the UI element for receiving calibration information from the user. If the error is resolved, the electronic apparatus (200) may reactivate the UI element. The UI element may be activated immediately after the blood glucose value normalizes or after a certain period of time. The UI element will be described in more detail with reference to FIG 5 below.

FIG 4 is a graph illustrating blood glucose rate of change according to an embodiment of the present disclosure. The graph (40) in FIG 4 corresponds to the graph (30) in FIG 3. The x-axis of the graph (40) represents time and the y-axis represents the blood glucose rate of change.

Referring to FIG 4, there may be a defined target range (TR) for the blood glucose range. The target range (TR) may be a criterion for determining whether there is an error in the blood glucose value. If the blood glucose rate of change is outside the target range (TR), the electronic apparatus (200) may determine that an error has been detected. In the graph (40), it can be seen that the blood glucose rate of change is outside the target range (TR) from 24:25 to 24:55. Therefore, the electronic apparatus (200) may determine that there is an error in the blood glucose value from 24:25 to 24:55.

The electronic apparatus (200) may perform the test each time a new sensor data or blood glucose value is acquired. Also, the electronic apparatus (200) may determine an error even if an abnormality in the blood glucose value does not necessarily persist for a preset time. For example, the electronic apparatus (200) may determine an error with just two consecutive blood glucose values. In the graph (40), the blood glucose rate of change at 24:25 may be calculated based on the blood glucose value at 24:20 and the blood glucose value at 24:25. If the blood glucose rate of change at 24:25 is outside the target range (TR), the electronic apparatus (200) may determine that an error has been detected without considering the blood glucose rate of change at 24:30.

FIG 5 is a diagram for explaining a blood glucose display screen according to an embodiment of the present disclosure.

Referring to FIG 5, the first screen (50-1) and the second screen (50-2) are blood glucose display screens displayed on the display (210) of the electronic apparatus (200). In the first screen (50-1), the UI element (52) is activated, and in the second screen (50-2), the UI element (52) is deactivated. The UI element (52) may be a component for receiving calibration information from the user. The user may select the activated UI element (52) and input the blood glucose value measured by the blood glucose meter as calibration information. When the UI element (52) is selected, the electronic apparatus (200) may display a calibration information input window.

If an error is detected as a result of the test on the blood glucose value, the electronic apparatus (200) may deactivate the UI element (52). At this time, the user cannot input calibration information through the UI element (52). For example, even if the user selects the UI element (52), the electronic apparatus (200) may not display the calibration information input window. If the error is resolved as a result of the test on the blood glucose value, the electronic apparatus (200) may reactivate the UI element (52). Accordingly, the user can select the UI element (52) to input calibration information.

The electronic apparatus (200) may deactivate the UI element (52) based on the user's sleep status. For example, if the user is sleeping, the electronic apparatus (200) may not deactivate the UI element (52) even if an error is detected. This is because the user does not select the UI element (52) while sleeping.

The UI element (52) may be displayed simultaneously with the blood glucose chart (51) on the same screen.

In one embodiment, the UI element (52) may be displayed overlaid on the blood glucose chart (51). In this case, the UI element (52) may cover a part of the blood glucose chart (51), making that part of the blood glucose value temporarily invisible. In particular, the UI element (52) may be positioned in an area where past blood glucose values are displayed, so as not to cover the part where the current blood glucose value is displayed in the blood glucose chart (51). This is to allow the user to always check the current blood glucose value while simultaneously being able to input calibration information.

In another embodiment, the UI element (52) may be displayed in an area that does not overlap with the blood glucose chart (51). If necessary, the user can move the UI element (52) or adjust its size to check the covered area of the blood glucose chart (51).

Meanwhile, in addition to the UI element (52), the blood glucose display screen may display a chart (51) indicating the blood glucose value, the current blood glucose value (53), and an indicator (54) indicating the change in blood glucose value.

According to various embodiments of the present disclosure, the electronic apparatus (200) may output a guide message about the user's behavior. For example, the guide message may instruct the user not to press the analyte monitoring device (100).

Additionally, if the user attempts to input calibration information while an error is detected, the electronic apparatus (200) may provide a guidance message such as "Calibration is not possible due to the unstable sensor state. Please try again after the sensor state stabilizes" along with deactivating the UI element (52). Such guide messages can help prevent mismeasurement due to inaccurate calibration and maintain the accuracy of analyte monitoring by informing the user of the current situation and appropriate response method.

The guide message may be provided not only in text format but also in various forms such as voice guidance, vibration patterns, or visual icons, and may be selectively activated according to the user's settings.

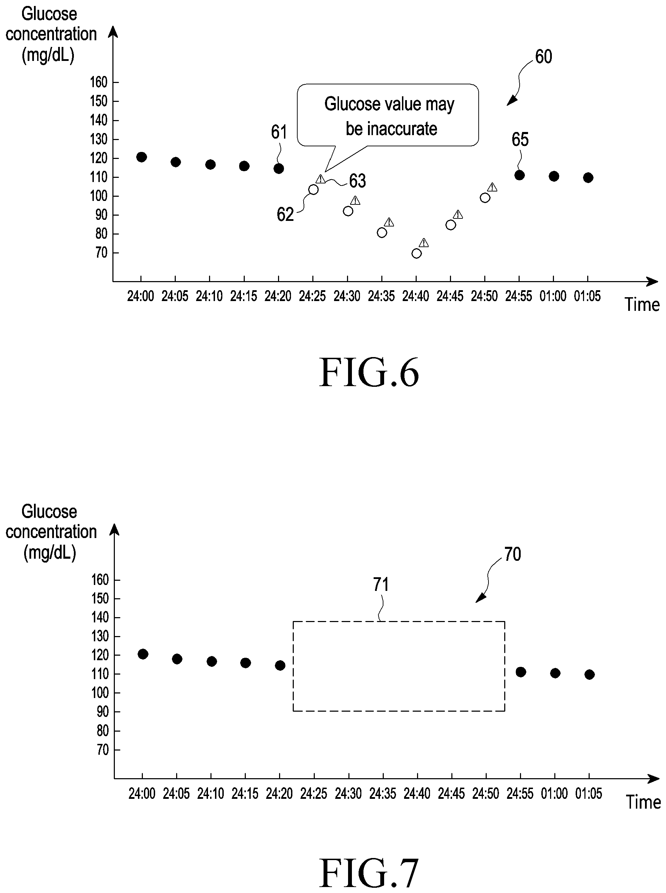

FIG 6 is a schematic diagram illustrating a blood glucose display method according to an embodiment of the present disclosure.

Referring to FIG 6, the blood glucose chart (60) displayed by the electronic apparatus (200) may show blood glucose values by time. The way blood glucose values are displayed may differ depending on whether an error is detected. For example, the first blood glucose value (61) may be a normal value with no error detected, and the second blood glucose value (62) may be an abnormal value with an error detected. In this case, the first blood glucose value (61) and the second blood glucose value (62) may be displayed differently. In one embodiment, the first blood glucose value (61) and the second blood glucose value (62) may be displayed with different shapes or colors. Also, a warning indicator (63) may be displayed around the second blood glucose value (62) to notify that the second blood glucose value (62) may be inaccurate. For example, if the user (1) selects the warning indicator (63), the electronic apparatus (200) may display a guidance message (64). In another example, the electronic apparatus (200) may display the guidance message (64) without the user's (1) selection.

The first blood glucose value (61) and the second blood glucose value (62) may be calculated in different ways. For example, the first blood glucose value (61) may be calculated based on sensor data and calibration information. The second blood glucose value (62) may be calculated based on normal blood glucose values at adjacent times. In one embodiment, the electronic apparatus (200) may generate the second blood glucose value (62) by performing interpolation based on the first blood glucose value (61) and the third blood glucose value (65). In another embodiment, the electronic apparatus (200) may generate the second blood glucose value (62) by performing extrapolation based on the first blood glucose value (61). Meanwhile, this is merely some embodiments, and the first blood glucose value (61) and the second blood glucose value (62) may be calculated in the same way.

FIG 7 is a schematic diagram illustrating a blood glucose display method according to another embodiment of the present disclosure.

Referring to FIG 7, the electronic apparatus (200) may not display blood glucose values in a time interval where an error exists (abbreviated as error interval) on the blood glucose chart (70). Therefore, a data gap (71) may be displayed on the blood glucose chart (70).

If an error is no longer detected, the electronic apparatus (200) may display blood glucose values in the error interval. That is, the electronic apparatus (200) may fill in the data gap (71) with blood glucose values. At this time, the blood glucose values displayed in the error interval may be calculated based on blood glucose values in the surrounding time intervals where no error exists. For example, the electronic apparatus (200) may calculate the blood glucose values in the error interval by performing interpolation or extrapolation on the surrounding blood glucose values.

According to another embodiment of the present disclosure, the electronic apparatus (200) may provide a predicted value of the analyte concentration to the user even when an error is detected. The electronic apparatus (200) may use various methods to predict the concentration of the analyte for a time interval where an error is detected in the sensor data.

In one embodiment, the electronic apparatus (200) may calculate a predicted value of the analyte concentration through time series analysis based on historical data. Specifically, the electronic apparatus (200) may analyze normal data for a certain period (e.g., the last 24 hours or the last 7 days) before the error is detected to identify the user's analyte concentration pattern. Through this, the average analyte concentration and change trends for specific time periods can be derived.

In another embodiment, the electronic apparatus (200) may perform more accurate predictions by learning the correlation between the user's behavioral information and analyte concentration. For example, if information about the user's lifestyle patterns, such as meal times, exercise times, and medication times, is recorded in the electronic apparatus (200), this information can be considered to calculate predicted values of analyte concentration. The electronic apparatus (200) may calculate expected changes in analyte concentration based on meal information (e.g., carbohydrate intake) input by the user.

In yet another embodiment, the electronic apparatus (200) may predict analyte concentration using machine learning algorithms. The electronic apparatus (200) may train a model to predict changes in analyte concentration using various factors as input values, such as the user's past analyte concentration data, lifestyle pattern information, and environmental factors. This model can be personalized for each user, allowing for more accurate prediction of analyte concentration change patterns for specific users.

The electronic apparatus (200) may display the predicted analyte concentration values in a way that is visually distinguishable from the actually measured analyte concentration values. For example, actual measured values may be displayed as solid lines, and predicted values as dotted lines. In addition, confidence intervals may be displayed around the predicted values to indicate the degree of uncertainty in the prediction. The confidence interval may vary depending on the accuracy of the prediction method, the amount of historical data available, and the temporal distance between the prediction point and the current point.

The electronic apparatus (200) may also display an icon or indicator along with the predicted values to indicate that they are predicted values. When the user selects this indicator, the electronic apparatus (200) may display a guidance message explaining how the predicted value was calculated, along with a notification that this value is a predicted value, not an actual measured value.

**In** one embodiment, when the error is resolved and the actual sensor data is received normally again, the electronic apparatus (200) may automatically replace the predicted values that were being displayed for that time interval with the actual measured values. **In** another embodiment, the electronic apparatus (200) may continue to display the predicted values or replace them with actual measured values according to the user's selection.

Additionally, the electronic apparatus (200) may continuously improve the prediction algorithm by analyzing the differences between predicted values and subsequently confirmed actual measured values. This allows for more accurate predicted values to be provided over time.

In this way, the electronic apparatus (200) according to the embodiment of the present disclosure can maintain the effect of continuous analyte monitoring by providing predicted values of analyte concentration as useful reference information to the user even when an error occurs in the sensor data. This may be particularly useful for users for whom continuous monitoring of analyte concentration is important, such as diabetic patients.

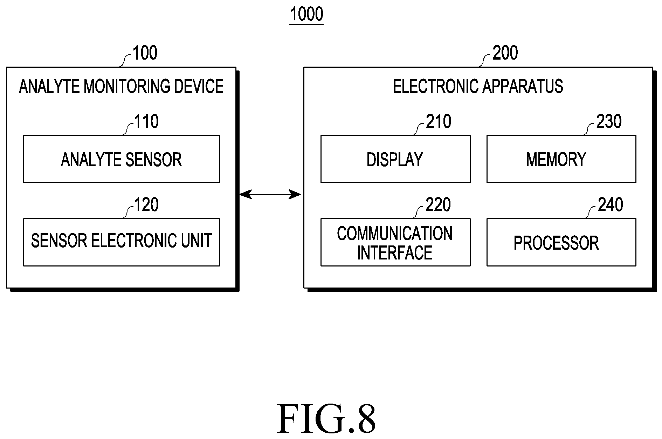

FIG 8 is a block diagram illustrating the configuration of an analyte monitoring system (1000) according to an embodiment of the present disclosure.

Referring to FIG 8, the analyte monitoring system (1000) may include an analyte monitoring device (100) and an electronic apparatus (200).

The analyte monitoring device (100) may include an analyte sensor (110) and a sensor electronic unit (120).

The analyte sensor (110) may be a component for sensing an analyte signal (or sensor signal). The analyte sensor (110) may include a sensor probe that is at least partially inserted into the body. A sensing area that reacts with glucose in the body to measure in-body glucose may be formed on the sensor probe.

The sensor electronic unit (120) may include at least one communication interface. The sensor electronic unit (120) may communicate with the electronic apparatus (200) through the communication interface. For example, the communication interface may include a Bluetooth module, a low power Bluetooth module, an RF module, and an NFC module.

The sensor electronic unit (120) may transmit sensor signals acquired through the analyte sensor (110) to the electronic apparatus (200). Also, the sensor electronic unit (120) may transmit the concentration of the analyte derived based on the sensor signal to the electronic apparatus (200). The sensor electronic unit (120) may transmit the sensor signal or the concentration of the analyte to the electronic apparatus (200) at a predefined period (e.g., 5 minutes).

The sensor electronic unit (120) may include an operating system (OS) for controlling the overall operation of the components of the analyte monitoring device (100) and a memory in which instructions or data related to the components of the analyte monitoring device (100) are stored. Additionally, the sensor electronic unit (120) may include a processor that is electrically connected to the memory and controls the overall functions and operations of the analyte monitoring device (100).

The electronic apparatus (200) may include a display (210), a communication interface (220), a memory (230), and a processor (240). For example, the electronic apparatus (200) may be a user terminal such as a smartphone or receiver.

The display (210) may output information related to the concentration of the analyte according to the control of the processor (240). For example, the display (210) may output the concentration of the analyte (e.g., blood glucose value) and guidance messages about the concentration of the analyte. The display (210) may include a touch screen. The user may input calibration information to calibrate the sensor data by touching the display (210).

The communication interface (220) may include at least one communication circuit. The communication interface (220) may receive sensor signals from the analyte monitoring device (100). The communication interface (220) may receive the concentration of the analyte from the analyte monitoring device (100).

The memory (230) may store an operating system (OS) for controlling the overall operation of the components of the electronic apparatus (200) and instructions or data related to the components of the electronic apparatus (200). The memory (230) may be implemented as a non-volatile memory (e.g., hard disk, Solid State Drive (SSD), flash memory) or a volatile memory.

The processor (240) may be electrically connected to the memory (230) and control the overall functions and operations of the electronic apparatus (200). The processor (240) may control the electronic apparatus (200) by executing instructions stored in the memory (230).

The processor (240) may receive sensor data from the analyte monitoring device (100) through the communication interface (230).

The processor (240) may control the display (210) to display the concentration of the analyte. The processor (240) may control the display (210) to display a UI element for receiving calibration information. The concentration of the analyte and the UI element may be displayed simultaneously. For example, the UI element may be overlaid on a chart containing the concentration of the analyte.

The processor (240) may receive calibration information through the UI element. The user may select the UI element through a user input unit to input calibration information. The user input unit may include a key pad, a dome switch, a touch pad (static/capacitive), a jog wheel, a jog switch, sensors (e.g., voice sensor, proximity sensor, illumination sensor, acceleration sensor, gyro sensor, etc.), and may be configured. The user input unit may be implemented in the form of buttons outside the electronic apparatus (200), and some buttons may be implemented as a touch panel.

The processor (240) may acquire the concentration of the analyte by calibrating the sensor data based on the calibration information. For example, the calibration information may include the user's blood glucose value measured through a blood glucose meter.

The processor (240) may detect an error by performing a test on the concentration of the analyte. For example, the processor (240) may detect whether the rate of change of the concentration of the analyte is outside a preset range. Also, the processor (240) may detect whether the rate of change of the concentration of the analyte is maintained below a threshold value for a preset period. Here, the threshold value may be negative.

The processor (240) may detect an error considering the user's sleep status. For example, if the user is not sleeping, the processor (240) may not detect an error.

The processor (240) may adjust the threshold value considering the user's sleep status. The threshold value may be negative, but may be set smaller when the user is not sleeping than when the user is sleeping. For example, when the user is not sleeping, the threshold value may be -3(mg/dL/min), and when the user is sleeping, the threshold value may be -2(mg/dL/min).

The processor (240) may not detect an error if the time point corresponding to the concentration of the analyte does not belong to a predetermined time zone. The predetermined time zone may correspond to the user's sleep time.

If an error is detected as a result of the test, the processor (240) may deactivate the UI element so that the user cannot input calibration information. If an error is not detected as a result of a subsequent test, the processor (240) may reactivate the UI element.

The processor (240) may control the display (210) to display the concentration of the analyte. The display (210) may display the concentration of the analyte differently according to the test result on the concentration of the analyte. For example, if an error is detected, the concentration of the analyte may be displayed in a first display mode, and if an error is not detected, the concentration of the analyte may be displayed in a second display mode.

The processor (240) may use blood glucose values from surrounding time intervals where no error is detected to calculate the blood glucose value for a time interval where an error is detected. For example, if an error is detected for a first concentration of the analyte corresponding to a first time point, the processor (240) may modify the first concentration based on a second concentration of the analyte corresponding to a second time point around the first time point.

The processor (240) may simultaneously display the UI element for receiving calibration information on the same screen as the concentration of the analyte.

According to an embodiment of the present disclosure, an error in a sensor signal can be detected.

According to an embodiment of the present disclosure, it is possible to prevent inaccurate blood glucose values from being provided to a user according to an error in the sensor signal. Accordingly, user convenience and satisfaction can be improved.

In addition to the effects obtained or predicted by the embodiments of the present disclosure, they are disclosed directly or implicitly in the detailed description of the embodiments of the present disclosure. For example, various effects predicted according to the embodiments of the present disclosure will be disclosed within the detailed description below.

The variety of embodiments described above can be implemented in a recording medium that can be read by a computer or a similar device using software, hardware, or a combination thereof. In some cases, the embodiments described herein may be implemented as a processor. When the embodiments are implemented as software, embodiments of procedures, functions, and the like described herein may be implemented as separate software modules. Each of the software modules may perform one or more functions and operations described herein.

Computer instructions for performing processing operations according to the variety of embodiments of the present disclosure described above may be stored in a non-transitory computer-readable recording medium. These computer instructions stored in the non-transitory computer-readable recording medium may cause a specific device to perform a processing operation according to the variety of embodiments described above when executed by a processor.

The non-transitory computer-readable medium is not a medium that stores data for a short moment, such as a register, a cache, a memory, or the like, but a machine-readable medium that stores data semi-permanently. Examples of the non-transitory computer-readable medium may include a compact disc (CD), a digital versatile disc (DVD), a hard disk, a Blu-ray disc, a Universal Serial Bus (USB) memory, a memory card, a read-only memory (ROM), and the like.

The machine-readable medium may be provided in the form of a non-transitory storage medium. Here, the "non-transitory storage medium" is a tangible device that does not include any signal (e.g., electromagnetic waves), and the term is used regardless of whether data is stored in a storage medium semi-permanently or temporarily. For example, the "non-transitory storage medium" may include a buffer in which data is temporarily stored.

Methods according to various embodiments disclosed in the present document may be included and provided in a computer program product. The computer program product may be traded as a product between a seller and a purchaser. The computer program product may be distributed in the form of a machine-readable storage medium (e.g., a CD-ROM) or distributed (e.g., downloaded or uploaded) online via an application store (e.g., PlayStore^{™}) or between two user devices (e.g., smart phones) directly. In the case of online distribution, at least a part of the computer program product (e.g., a downloadable app) may be temporarily generated or at least temporarily stored in a machine-readable storage medium such as memory of a manufacturer's server a server of the application store, or a relay server.

Although exemplary embodiments of the present disclosure have been illustrated and described above, the present disclosure is not limited thereto. Those of ordinary skill in the art can make various modified embodiments without departing from the gist of the present disclosure described in the claims, and it is to be understood that these modified embodiments fall within the technical spirit or scope of the present disclosure.

## Claims

1. A method for controlling an electronic apparatus, comprising:
receiving a sensor data related to a concentration of an analyte from an analyte monitoring device at least partially implantable beneath a skin of a user;
displaying a UI element for receiving a calibration information from the user, wherein the concentration of the analyte is acquired based on the sensor data and the calibration information; and
deactivating the UI element when an error related to the sensor data is detected.

2. The method of claim 1, wherein the error is detected when a rate of change of the concentration of the analyte is outside a preset range.

3. The method of claim 1, wherein the error is detected when a rate of change of the concentration of the analyte remains below a threshold value for a preset period.

4. The method of claim 3, wherein the threshold value is adjusted based on whether the user is sleeping.

5. The method of claim 1, wherein the error is not detected when the user is not sleeping.

6. The method of claim 1, wherein the error is not detected when a time point corresponding to the concentration of the analyte does not belong to a predetermined time period.

7. The method of claim 1, further comprising: acquiring the concentration of the analyte by calibrating the sensor data based on the calibration information when the calibration information is received from the user.

8. The method of claim 1, wherein the concentration of the analyte is displayed in a first display mode when the error is detected, and the concentration of the analyte is displayed in a second display mode different from the first display mode when the error is not detected.

9. The method of claim 1, further comprising: modifying a first concentration of the analyte corresponding to a first time point based on a second concentration of the analyte corresponding to a second time point around the first time point, when an error related to a first sensor data corresponding to the first concentration of the analyte is detected.

10. The method of claim 1, wherein the UI element is displayed simultaneously with the concentration of the analyte on the same screen.

11. An electronic apparatus, comprising:
a display;
a communication interface including at least one communication circuit;
a memory storing at least one instruction; and
a processor;
wherein the processor, by executing the at least one instruction,
receives a sensor data related to a concentration of an analyte from an analyte monitoring device at least partially implantable beneath a skin of a user,
displays a UI element on the display for receiving a calibration information from the user, wherein the concentration of the analyte is acquired based on the sensor data and the calibration information is used for calibrating the sensor data, and
deactivates the UI element when an error related to the concentration of the analyte is detected.

12. The electronic apparatus of claim 11, wherein the processor detects the error when a rate of change of the concentration of the analyte is outside a preset range.

13. The electronic apparatus of claim 11, wherein the processor detects the error when a rate of change of the concentration of the analyte remains below a threshold value for a preset period.

14. The electronic apparatus of claim 13, wherein the processor adjusts the threshold value based on whether the user is sleeping.

15. The electronic apparatus of claim 11, wherein the processor does not detect the error when the user is not sleeping.

16. The electronic apparatus of claim 11, wherein the processor does not detect the error when a time point corresponding to the concentration of the analyte does not belong to a predetermined time period.

17. The electronic apparatus of claim 11, wherein the processor acquires the concentration of the analyte by calibrating the sensor data based on the calibration information when the calibration information is received from the user.

18. The electronic apparatus of claim 11, wherein
the processor displays the concentration of the analyte in a first display mode when the error is detected, and
displays the concentration of the analyte in a second display mode different from the first display mode when the error is not detected.

19. The electronic apparatus of claim 11, wherein the processor modifies a first concentration of the analyte corresponding to a first time point based on a second concentration of the analyte corresponding to a second time point around the first time point, when an error related to the first concentration of the analyte is detected.

20. The electronic apparatus of claim 11, wherein the processor displays the UI element simultaneously with the concentration of the analyte on the same screen.
